# EUROPEAN PATENT APPLICATION

(11) **EP 1 310 551 A1**
(43) Date of publication of application: **14.05.2003**
(21) Application number: 01954500.3
(22) Date of filing: 07.08.2001
(51) Int. Cl.: C12N 7/00, C12N 7/02, C12N 15/33, A61K 38/00, A61K 39/21, A61K 45/00, A61K 48/00, A61P 31/12

(54) **VIRUS-LIKE MICROGRAINS AND PROCESS FOR PRODUCING THE SAME**

(30) Priority: 08.08.2000 JP 2000239903
(71) Applicant: THE KITASATO INSTITUTE, Tokyo 108-8641 (JP)
(72) Inventor: MORIKAWA, Yuko, Inagi-shi, Tokyo 206-0802 (JP)
(74) Representative: Woods, Geoffrey Corlett
(86) International application number: JP0106791
(87) International publication number: WO02012454

(57) **Abstract**

The present invention provides a process for producing virus-like particles, which can produce virus-like particles having the protein of a virus and a lipid bilayer membrane derived from an eukaryotic microorganism as an outer membrane in a short period of time in a form that is not contaminated with a vector virus, and which enables the virus-like particles to be used for various applications. The process of the present invention comprises:
(a) incorporating a gene encoding a protein essential for viral particle budding or a fragment thereof into a vector which can be expressed in a eukaryotic microorganism;
(b) transfecting the vector into the eukaryotic microorganism;
(c) culturing a transformed eukaryotic microorganism;
(d) removing a cell wall of the eukaryotic microorganism; and
(e) further culturing, followed by collection of a culture supernatant.

## Description

### Technical Field

The present invention relates to virus-like particles and a process for producing the same. More particularly, the present invention relates to virus-like particles having a lipid bilayer membrane derived from a eukaryotic microorganism as an outer membrane, a process for producing the same, and an application thereof.

### Prior Art

Viruses can be roughly categorized, in accordance with their forms, into non-enveloped viruses (viruses without a lipid bilayer membrane) and enveloped viruses (viruses with a lipid bilayer membrane).

A life cycle of a virus proceeds from an early stage (adsorption, entry), to an intermediary stage (transcription, replication), and then to a late stage (protein synthesis, assembly). In order to identify a host factor required in these stages, the use of yeast cells, which allow modification of a host gene, has been recently attempted.

The formation of virus particles of enveloped viruses is completed in cytoplasm of the host cells, and their progeny viruses are released upon disruption of the host cells. Accordingly, virus-like particles (VLP) of such viruses, when reproduced in the yeast cells, are formed in the cytoplasm, and the VLP are purified by deliberate disruption of yeast cells (Valenzuela, P. et al., 1982, Nature 298: 347-350; Cook, J. C. et al., 1999, Protein Expr. Purif. 17: 477-484; Jore, J. P. et al., 1994, Yeast 10: 907-922). It has been actually elucidated that the majority of a life cycle of animal viruses parasitizing higher eukaryotic cells, for example, hepatitis B virus, human papilloma virus, polio virus, and polyoma virus, can be reproduced in yeast cells as a host: the early stage (Makarow, M. 1985, EMBO J. 4: 1855-1860; Makarow, M. et al., 1987, J. Biol. Chem. 262: 1836-1841), the intermediary stage (Janda, M. and Ahlquist, P. 1993, Cell 72: 961-970; Price, B. D. et al., 1996, Proc. Natl. Acad. Sci. USA 93: 9465-9470), the late stage (Price, B. D. et al., 1996, Proc. Natl. Acad. Sci. USA 93: 9465-9470; Valenzuela, P. et al., 1982, Nature 298: 347-350).

The life cycle of non-enveloped viruses is completed through these 3 stages, but, in contrast, the life cycle of enveloped viruses is completed by a final assembly stage termed budding, which leads to assembly of viral components on the cell membrane and release of viral particles enclosed in the lipid membrane into intracellular vesicles(endoplasmic reticulum or Golgi apparatus) or from the plasma membrane. At present, there is no report in which this budding process was successfully reproduced using yeast cells as a host.

What is reported is the production of virus-like particles for enveloped viruses, by using an established cell line of the higher eukaryotic cells which express their virus proteins. This system, however, has drawbacks, for example, it takes 2 to 3 months to establish such cell lines and to produce the particles, and the yield of particles produced is low. Further, in producing biologicals for humans using this system, production is limited to the use of licensed cell lines (e.g., Vero cell).

Also reported is a system for producing virus-like particles using a virus vector and higher eukaryotic cell as a host, for example, a recombinant *baculovirus* expression system or a recombinant vaccinia virus expression system. These systems can provide particles in a shorter period of time compared to the system of an established cell line of the higher eukaryotic cells. However, the use thereof was restricted from the viewpoint of safety due to a fear of contamination with the virus vector used for the expression, many of which are infectious.

### Disclosure of the Invention

Heretofore, there has been no method, without contamination of a vector virus, which can produce the virus-like particles having the protein of the virus with a lipid bilayer membrane as an outer membrane in a short period of time, and which enables the virus-like particles to be used for various applications.

The virus protein necessary for virus particle budding with a lipid bilayer membrane varies depending on the type of virus. Garoff et al. (Garoff, H. et al., 1998, Microbiol. Mol. Biol. Rev. 62: 1171-1190) reviewed studies of the minimal units for particle budding for many viruses, and categorized into 4 types. These categories are: type I) which requires both capsid protein and spike protein (*alphavirus* and *hepadonavirus*); type II) which requires only capsid protein (*retrovirus*); type III) which requires only membrane/matrix protein (*coronavirus*); and type IV) which requires spike protein and RNP in addition to membrane/matrix protein (*rhabdovirus, paramyxovirus*, and *orthomyxovirus*).

In the case of the human immunodeficiency virus (HIV) belonging to *retrovirus,* for example, its particle formation is found to be driven by the Gag protein, which is the major structural protein. In higher eukaryotic cells, virus-like particles (VLP) are found to be produced by the expression of the Gag protein alone (Smith, A. J. et al., 1993. J. Virol. 67: 2266-2275; Gheysen, D. et al., 1989, Cell 59: 103-112).

Morphogenesis of these particles goes through processes of: 1) myristoylation at the N terminus of the Gag protein in the cytosol; 2) myristoylation-dependent targeting to the plasma membrane; 3) assembling (i.e., multimer formation) underneath the plasma membrane; and 4) particle formation through budding. However, in any conventional system using a lower eukaryotic cell, i.e., yeast, morphogenesis went no further than step (3). Specifically, particle budding did not take place although the myristoylated Gag protein targeted the plasma membrane.

We have conducted concentrated studies in view of the above circumstances, and as a result, we have found that by removing the cell wall from an eukaryotic microorganism such as yeast, which has been transfected with the expression plasmid having HIV-derived Gag gene incorporated therein, VLP enclosed with a microorganism-derived lipid bilayer membrane was spontaneously budded and released in a very short period of time.. This method can be applied to HIV as well as a variety of enveloped viruses . The particle budding and releasing system according to the present invention is promising as a vector virus-free production system for VLP vaccine (antigen with defined and particulate structure) that has immunogenicity markedly higher than that of a component vaccine (comprising a soluble protein as an antigen).

More specifically, the present invention provides the following (1) to (13).
(1) Virus-like particles having a lipid bilayer membrane derived from a eukaryotic microorganism as an outer membrane.
(2) The virus-like particles according to (1) above, wherein the eukaryotic microorganism is yeast.
(3) The virus-like particles according to (1) or (2) above, which have immunogenicity and do not produce infectious progeny viruses.
(4) The virus-like particles according to any one of (1) to (3) above, wherein the immunogenicity derives from HIV.
(5) A process for producing virus-like particles, comprising:
   (a) incorporating a gene encoding a protein essential for virus particle budding or a fragment thereof into a vector which can be expressed in a eukaryotic microorganism;
   (b) transfecting the vector into the eukaryotic microorganism;
   (c) culturing a transformed eukaryotic microorganism;
   (d) removing a cell wall of the eukaryotic microorganism; and
   (e) further culturing, followed by collection of a culture supernatant.
(6) The process according to (5) above, wherein the gene or a fragment thereof comprises a gene encoding viral capsid protein or membrane/matrix protein or a fragment thereof.
(7) The process according to (5) above, wherein the gene to be incorporated comprises a gene encoding a protein associated with immunogenicity or a fragment thereof.
(8) The process according to any of (5) to (7) above, wherein the eukaryotic microorganism is yeast.
(9) The process according to any of (5) to (8) above, wherein the virus is HIV.
(10) A pharmaceutical composition, which comprises the virus-like particles according to any of (1) to (4) above.
(11) The pharmaceutical composition according to (10) above, which is a vaccine having immunogenicity derived from an incorporated virus gene.
(12) The pharmaceutical composition according to (10) above, which comprises a virus-derived protein and at least one active ingredient
(13) The pharmaceutical composition according to (12) above, wherein the active ingredient is virus-derived nucleic acid, ribozyme, antisense nucleic acid, protein or a fragment thereof, or physiologically active protein or a fragment thereof.

This specification includes part or all of the contents as disclosed in the specification and/or drawings of Japanese Patent Application No. 2000-239903, which is a priority document of the present application.

### Brief Description of Drawings

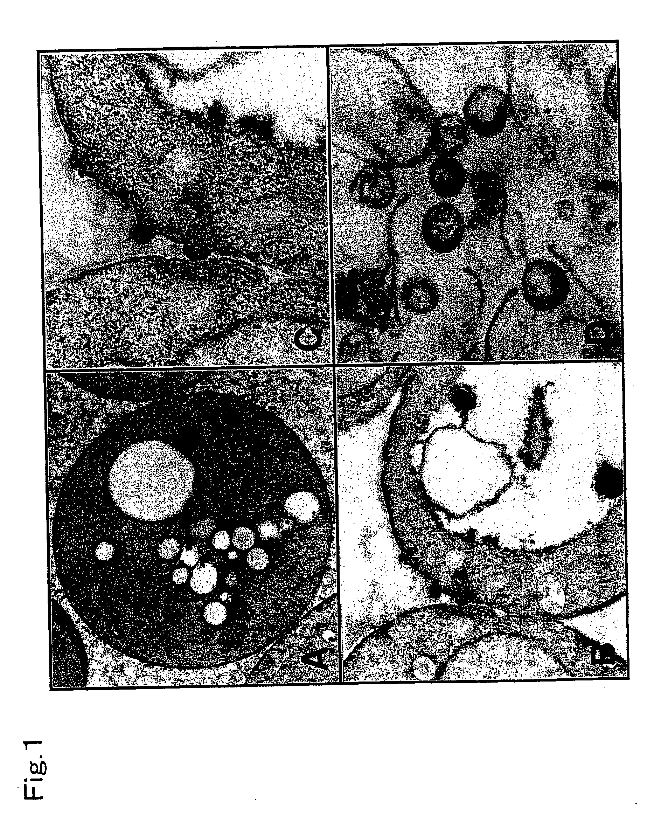

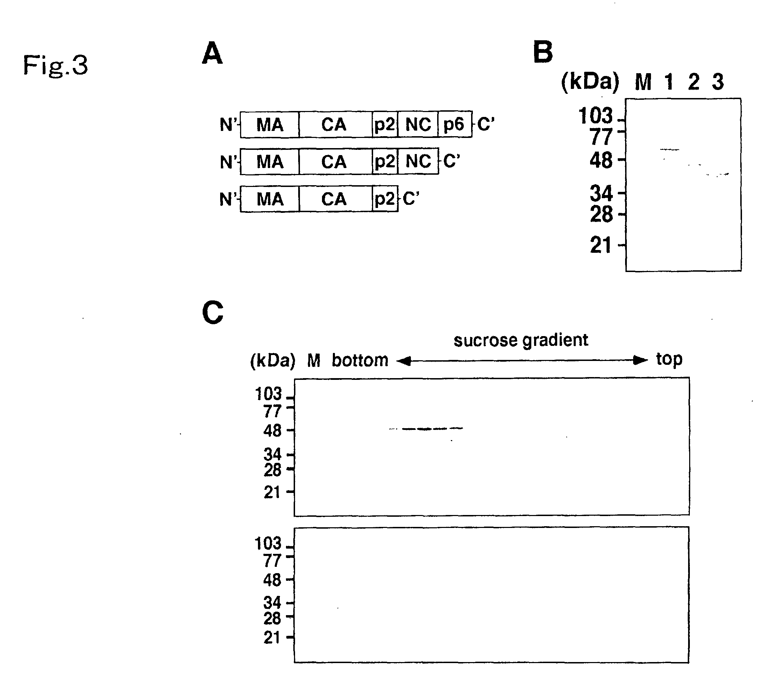

Fig. 1 shows electron micrographs of a yeast spheroplast cell expressing HIV-1 Gag protein and purified VLP, wherein
   (A) shows the yeast spheroplast for Gag protein expression immediately after the removal of the cell wall,
   (B) shows the yeast spheroplast for Gag protein expression which was cultured for 2 hours after removal of the cell wall,
   (C) shows a partially enlarged photograph of (B), and
   (D) shows the purified VLP obtained after being collected from the culture supematant.

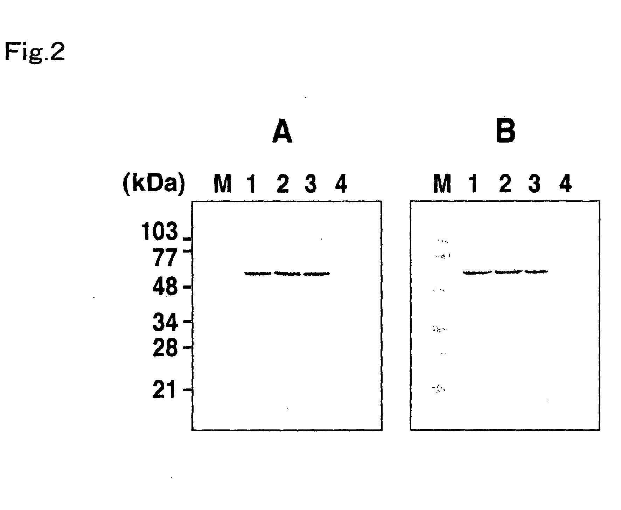

Fig. 2 shows the result of the protein detection by Western blotting after Triton X-100 treatment and trypsin digestion of the purified VLP, wherein
   (A) shows the VLP purified from the culture supematant of the yeast spheroplast cells expressing HIV-1 Gag protein, and
   (B) shows the VLP purified from the culture supernatant of insect Sf9 cells, which were expressed HIV-1 Gag protein using a recombinant *baculovirus*.
      M) molecular weight marker,
      1) Not treated,
      2) Treated with Triton X-100,
      3) Treated with trypsin,
      4) Treated with Triton X-100 and trypsin
Fig. 3 shows an effect of the domain truncation from the C terminus of the HIV-1 Gag protein on the VLP production. In Fig. 3,
   (A) shows a domain structure of the wild type Gag protein and the truncation mutants.
   (B) shows the result of the detection of Gag protein in the transformed yeast cells by Western blotting.
      M) a molecular weight marker,
      1) the yeast cells expressing wild type Gag protein,
      2) the yeast cells expressing MA-CA-p2-NC construct,
      3) the yeast cells expressing MA-CA-p2 construct
   (C) shows the VLP purified from the culture supernatant of the transformed yeast spheroplasts.
      M) a molecular weight marker, (upper level) the VLP from the culture supernatant of the yeast expressing MA-CA-p2-NC, (lower level) the VLP from the culture supernatant of the yeast expressing MA-CA-p2 .
Fig. 4 shows the yield kinetics of VLP production with time, wherein
   (A) shows the result of Western blotting on the VLP fraction at various time points of culture, and
   (B) shows the quantitated level of VLP produced per liter of spheroplast culture medium at the corresponding time point of culture.
Fig. 5 shows the protein detection by Western blotting of the VLP purified from the yeast spheroplast cells expressing HIV-1 Gag-V3 protein in which
   M) a molecular weight marker,
   1) the Gag antigen detected with an anti-HIV-1 CA monoclonal antibody,
   2) the V3 antigen detected with an anti-HIV-1 V3 monoclonal antibody having a neutralizing activity.

### Preferred Embodiments of the Invention

As described above, the present invention provides virus-like particles having a lipid bilayer membrane derived from a eukaryotic microorganism as an outer membrane.

Examples of the eukaryotic microorganism which can be used in the present invention include yeast, fungal cell, algal cell, and protozoan. The use of yeast is particularly preferred because genetic engineering can be fully utilized, more specifically, a wide variety of expression vectors are already available, the genomic analysis has been completed, and the use thereof in the production of foods and vaccines has been practically accomplished.

In the present invention, the term "virus-like particles" refers to deficient virus particles which cannot produce infectious progeny virus due to deficiency or mutation in virus genes.

Viruses to which the present invention can be applied are not particularly limited as long as the virus has a lipid bilayer membrane. Either DNA virus or RNA virus may be used, however, RNA virus is preferred since DNA virus with a lipid bilayer membrane has genes encoding many proteins, and the functions of all the proteins are not yet known. Further, the virus-like particles obtained in the present invention should not produce infectious progeny viruses. Thus, preferable viruses are those that do not have effective and safe attenuated virus strains which can be used as a live vaccine, and from which the production of noninfectious virus particles can be expected. Specific examples of such viruses include immunodeficiency virus, Ebola and Marburg virus, influenza virus, dengue virus, and Japanese encephalitis virus.

The virus-like particles contain all or some of viral structural proteins which all or some of the virus-derived structural genes has been expressed in the eukaryotic microorganism. Examples of the viral proteins that constitute the virus-like particles include capsid protein, spike protein, membrane/matrix protein, and core protein. In order to produce and release virus-like particles, it is necessary to contain a virus protein that is necessary for budding and that has affinity to the host cell membrane, for example, capsid protein such as the HIV Gag protein, membrane/matrix protein such as M protein in measles virus or influenza virus, and the like. The minimal unit for budding for individual viruses is described in Garoff et al. (Garoff, H. et al., 1998, Microbiol. Mol. Biol. Rev. 62: 1171-1190).

Other proteins necessary as proteins contained in virus-like particles can vary depending on the application of the virus-like particles according to the present invention. For example, when the virus-like particles of the present invention are used as the vaccine antigen for the virus, a protein region for determining the immunogenicity of the virus, for example, a neutralizing epitope, Th epitope, or CTL epitope, is preferably contained. In contrast, when the virus-like particles of the present invention are used as an adjuvant by, for example, including other antigens, or when the virus-like particles of the present invention are used as a carrier of a pharmaceutical composition comprising another active ingredient, a protein region for determining the immunogenicity of the virus itself is preferably not contained.

The present invention also provides a process for producing virus-like particles comprising:
(a) incorporating a gene encoding a virus-derived protein essential for the budding of virus-like particles or a fragment thereof into a vector which can be expressed in an eukaryotic microorganism;
(b) transfecting the vector into the eukaryotic microorganism;
(c) culturing a transformed eukaryotic microorganism;
(d) removing the cell wall of the eukaryotic microorganism; and
(e) further culturing, followed by collection of the culture supematant.

Virus-derived genes to be expressed in an eukaryotic microorganism are not particularly limited. As mentioned above, however, a gene encoding a protein that is necessary for budding and that has affinity to a host cell membrane or a fragment thereof, i.e., at least a gene encoding capsid protein or membrane/matrix protein or a fragment thereof, should be contained.

Vectors which can be used in the present invention are not particularly limited as long as they are used in the art, and examples thereof include pKT10 and pYES2, which are suitable as the expression vector in the eukaryotic microorganism. Expression vectors preferably include: a regulatory sequences for protein expression such as promoter, enhancer, and terminator; a replication origin; and a selection marker such as URA3, LEU2, HIS3, TRP1, and LYS2.

A method for transfecting the vector into the eukaryotic microorganism is well known in the art, and examples thereof include, but are not limited to, electroporation and the lithium acetate method.

Transformation and protein expression can be confirmed by observing the expression of the transfected gene by binding of the specific antibody against the expressible protein, such as Western blotting, or ELISA, as commonly performed in the art.

Culture conditions for the host eukaryotic microorganism vary depending on a type of microorganism to be selected. For example, in the case of yeast, culture is preferably conducted at 25 to 37°C, normally at 30°C, under shake culture conditions. A person skilled in the art can easily conceive of suitable culture conditions for each selected host microorganism.

Removal of a cell wall, which is a necessary step in the method of the present invention, can be carried out by, for example, digesting the cell wall using a cell wall digestion enzyme such as Zymolyase and removing the digested cell wall pieces by washing and the like.

Immediately after the removal of the cell wall, the virus-like particles of the present invention begin to be released in the culture supernatant. The particles can be collected from the culture supernatant by first centrifuging the culture supernatant to remove the cells and debris, and subsequently by ultracentrifuging the supernatant on a sucrose gradient, although the collection method is not limited to this. Since the virus-like particles of the present invention are released from the host eukaryotic microorganism by budding, separation from the microorganism is very easy and purification can be easily carried out.

Genes incorporated by the method of the present invention comprise a gene encoding a viral protein or a fragment thereof that is necessary for particle budding of virus and that has affinity to a host cell membrane, i.e., at least one gene including a gene which encodes capsid protein or membrane/matrix protein, or a fragment thereof. In this specification, the term "fragment" refers to a portion of a specific gene, and a protein, which is obtained by the expression of the partial gene, has a function similar to that of a protein obtained by the expression of a full-length gene. A person skilled in the art can suitably determine the type and range of genes to be incorporated according to the application of interest.

Types of genes to be incorporated vary according to the applications of the virus-like particles obtained. When the virus-like particles are used as the vaccine antigen for the virus, a protein region associated with the immunogenicity of the virus is preferably contained. In contrast, when the virus-like particles of the present invention are used as microcapsulated carrier particles by, for example, having another active ingredient encapsulated, a protein region for determining the immunogenicity of the virus itself is preferably not contained.

Viruses that can be used in the method of the present invention are not particularly limited. Regarding yeast cells in which Gag protein of human immunodeficiency virus (HIV) is expressed, for example, it is known that, with a conventional technique, particle formation and budding did not take place, although the Gag protein targeted to the plasma membrane. In the present invention, however, when the cell wall was removed from the yeast expressing Gag protein and then cultured, Gag virus-like particles (VLP) were spontaneously budded and released into the culture medium. The HIV VLP which was budded from the yeast had the following features:
1) the HIV VLP was morphologically very close to VLP obtained by Gag expression in higher eukaryotic cells;
2) the particles were in the form of being completely enveloped in a lipid bilayer membrane as an outer membrane of the particles;
3) the particles began to be released immediately after removal of the cell wall and the level produced increased with time; and
4) a region necessary for the VLP production mapped from the result of the truncation experiment on the Gag protein is identical with the necessary region mapped in the VLP production in higher eukaryotic cells.

Accordingly, use of the system for budding and releasing VLP from the eukaryotic microorganism including yeast cells according to the present invention can be expected as a vector virus-free system for VLP production.

As described above, we elucidated that, upon removal of the cell wall from yeast cells in which Gag protein of HTV, which is an enveloped virus, has been constitutively expressed, the Gag VLP, which has a lipid bilayer membrane as an outer membrane, is spontaneously budded and released, and can be purified from the culture medium.

This system for budding and releasing VLP from the yeast spheroplast is a powerful tool for production of biologicals.

In general, a system for producing a high level of VLP is an expression system of higher eukaryotic cells using a virus as an expression vector (e.g., a recombinant *baculovirus* expression system). However, VLP fractions obtained by such a system are contaminated with the virus that was used as the expression vector itself and inappropriate for administration to humans. In contrast, the system for budding and releasing VLP from the yeast spheroplast that was developed in the present invention is a vector virus-free system for producing VLP. Also, since the expression system is plasmid-based, the preparation thereof is much simpler and easier compared to that of the recombinant virus-based system. Accordingly, the production of VLP, in order to cope with mutation of virus antigen and diversity of antigen subtypes, whose viral protein is replaced with the mutant protein or the individual subtype, can be rapidly carried out. Further, the production of VLP for personal use, which was difficult in the past, can be also carried out. More specifically, for example, when mutation of virus antigen occurs in the body of a patient infected with a virus such as HIV, a virus gene can be separated from a sample obtained from the patient and genetic engineering techniques can be employed to artificially modify the gene in order to prevent infectious progeny viruses from being produced, for example, by causing functional defects of a part of the gene encoding the Pol protein or the LTR region. Thus, the virus-like particles produced according to the method of the present invention can be used as virus-like particles or booster antigen which can specifically be administered to the individual patient.

Advantages and disadvantages of the present VLP budding system and the conventional VLP budding system (recombinant *baculovirus* expression system and higher eukaryotic cell line established for VLP budding) were compared in order to clarify the features of the method according to the present invention (Table 1).

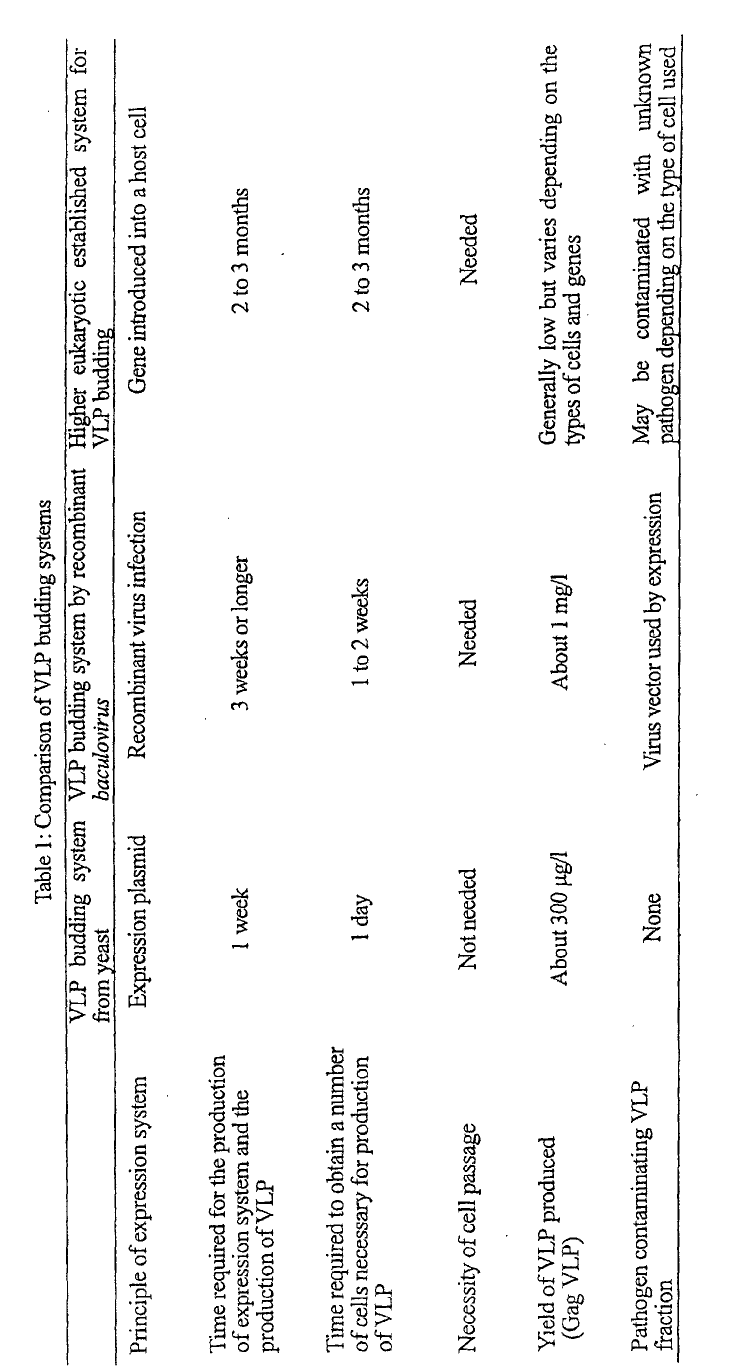

As is apparent from Table 1, the budding system according to the present invention requires a very short period of time, i.e., 1 week, for the establishment of the expression system and the production of VLP. Also, due to the high proliferation capacity, it takes only 1 day to obtain the necessary number of cells, and VLP yield is also high. Further, since passages or maintenance of cells is not required, operation is accompanied by time labor saving. Also, cell culture can be carried out in a cost-effective and simple manner. It is a particularly important advantage that there is no contamination by pathogens. Furthermore, genetic manipulations can be easily carried out, and it can be applied to viruses which are less infectious to higher animals or plant cells.

In contrast, the establishment of a cell line, using higher eukaryotic cells, which expresses viral protein requires a very long period of time, i.e., 2 to 3 months, to produce the expression system and the like. Also, passages or maintenance of cells is required, and it is impossible to rapidly establish an altemate cell line to cope with virus mutation. The system using recombinant *baculovirus* requires a somewhat shorter time for the production of the expression system and the like compared to the system using the established cell line of higher eukaryotic cells. Passages or maintenance of cells, however, is required, and the virus vector itself used for the expression contaminates the system. Thus, its application is disadvantageously limited.

Accordingly, the virus-like particles obtained by the method of the present invention can be applied to a wide variety of purposes by virtue of the features.

The present invention, therefore, provides a pharmaceutical composition comprising the virus-like particles.

One example of the pharmaceutical composition of the present invention is a particulate vaccine having immunogenicity derived from a part of the virus gene incorporated into the expression vector . Specifically, the virus-like particles according to the present invention have the same immunogenicity as the origin virus and function as a vaccine by administration to a human. In this case, the proliferative capacity or pathogenicity of the virus used for VLP construction is preferably attenuated or eliminated by genetic manipulations and the like. The virus-like particles of the present invention can be easily purified from the host cell culture and can be obtained in a short period of time. Thus, they can be rapidly prepared according to the need, for example, when prevalence of the virus infection occurs. Because the particles have a lipid bilayer membrane as an outer membrane, the encased protein inside of the particle is stable in blood stream, as with liposome, which is known as an effective carrier. Compared to the component viral antigen which is soluble protein, the viral protein forming a particle exhibits the authentic defined structure of the virus and is closer to the native form of the viral antigen, suggesting higher immunoinduction can be expected. Further, the adjuvant effect can also be expected due to the presence of the lipid bilayer membrane. Furthermore, a safe vaccine which is vector virus-free and noninfectious can be provided. As described above, particles for personal use which are available for an individual patient can be prepared.

The present invention can also provide a pharmaceutical composition comprising at least 1 active ingredient together with a virus-derived protein. Examples of the active ingredient include virus-derived nucleic acid, ribozyme, antisense nucleic acid, protein or a fragment thereof, and other various pharmaceuticals. In this specification, the term "fragment" refers to a portion of a specific gene, and a protein expressed by the partial gene has a function similar to that of a protein expressed by a full-length gene. A person skilled in the art can suitably determine the type and length of genes to be incorporated according to the application of interest. The embodiment of the pharmaceutical composition according to the present invention is not particularly limited. Examples thereof include: DNA vaccines encased in a lipid bilayer membrane; particles enveloping antiviral agents, such as a ribozyme for cleaving the virus gene in the infected cell or an antisense nucleic acid against the virus gene; microcapsulated carrier particles enveloping antibodies, enzymes, dominant negative virus proteins, other functional proteins, and antibiotics.,. These active ingredients can be suitably incorporated into the composition during the genetic engineering or producing process for the virus-like particles. In this embodiment, the virus-like particles of the present invention are expected to function as a carrier for the active ingredient and, optionally, to exhibit the immunity enhancing effect.

The pharmaceutical composition of the present invention comprises the virus-like particles which may contain at least one active ingredient as an essential component. In addition, it may be suitably combined with a pharmaceutically acceptable carrier or medium to be formulated and administered. Specific examples thereof include sterilized water or physiological saline, vegetable oil, emulsifier, suspension, surfactant, stabilizer, binder, lubricant, sweetener, flavoring agent, and coloring agent. Administration to a human can be carried out by various commonly-used methods, for example, intravenous, intraarterial, subcutaneous, or intramuscular injection or infusion, or intranasal, transbronchial, or oral administration. The dose varies depending on, for example, the body weight and age of the individual and the route of administration, and a person skilled in the art can suitably select an adequate dose.

### EXAMPLES

The present invention will be described in more detail with reference to the following examples, although it is not limited to these examples.

### [Example 1] Budding and releasing of HIV VLP from yeast spheroplast cell

A cDNA fragment encoding HIV Gag protein (full-length, MA-CA-p2-NC or MA-CA-p2) was inserted into YEp type vector pKT10 having URA3 for a selection marker and GAP promoter for constitutive expression (Tanaka, K. et al., 1990, Mol. Cell. Biol. 10: 4303-13) to construct a Gag protein-expression vector. The vector was introduced into RAY3A-D strain (MATa/α ura3/ura3 his3/his3 leu2/leu2 trp1/trp1) (Ruggieri, R. et al., 1989, Proc. Natl. Acad. Sci. USA 86: 8778-8782) of yeast (*Saccharomyces cerevisiae*) by electroporation. The yeast cells were grown on a uracil selective plate (0.67% yeast nitrogen base, 2% glucose, uracil-free amino acid mixture, 2% agar), and the formed colonies were selected as transformed cells. A lysate of this transformed yeast cells were analyzed by SDS-PAGE and Western blotting (Towbin, H. et al., 1979, Proc. Natl. Acad. Sci. USA 76: 4350-4354) using anti HIV-1 CA monoclonal antibody (Advanced Biotechnologies Inc.). As a result, it was confirmed that yeast cells which express the full-length Gag protein were obtained.

The transformed yeast cells were grown in a uracil-free completely synthetic medium (0.67% yeast nitrogen base, 2% glucose, uracil-free amino acid mixture) until OD 600 reaches 2.0 to 2.5. Culture was conducted at 30°C. Subsequently, in order to prepare yeast spheroplasts , cells were washed with a washing buffer (50 mM Tris [pH 7.5], 5 mM MgCl₂, 1 M sorbitol), suspended in a washing buffer containing 30 mM DTT and gently shaken at 30°C for 20 minutes. To remove the cell wall, the cells were resuspended in a washing buffer containing 3 mM DTT, and Zymolyase-100T (SEIKAGAKU CORPORATION) was added to a final concentration of 0.4 mg/ml. For the digestion reaction, the cells were subjected to soft shake culture at 30°C for 20 minutes. At this stage, whether or not the cell wall of the yeast was removed was determined. Specifically, the removal of the cell wall was confirmed by observation that the cell was disrupted in a hypotonic solution due to the absence of the cell wall, and a part of the cell suspension was taken and resuspended in water.. After confirmation, the cells were washed with an isotonic solution of 1 M sorbitol, and cultured in a YPD medium supplemented with 1 M sorbitol. In accordance with conventional techniques (Goto, T. et al., 1990, Arch. Virol. 111: 87-101), the electron microscopic observation was carried out following fixing, staining, and slicing the sample.

After the cell wall was removed from yeast, culture was conducted for 2 hours. Electron microscopic analysis revealed that the spheroplast cells exhibit a structure which was not observed immediately after the cell wall removal was observed, i.e., there were several images of budding with an electron-dense submembrane layer from a plasma membrane (Fig. 1A, B, C).

VLP was purified from the culture medium of the obtained yeast spheroplasts. At the outset, the culture medium was centrifuged (7,000 rpm, 30 minutes) to remove the cells and the debris, and the supernatant was applied onto a 40% sucrose/PBS, followed by ultracentrifugation (24,000 rpm, 90 minutes). The pellet was suspended in PBS and applied onto a 20 to 70% sucrose/PBS gradient, followed by ultracentrifugation (35,000 rpm, overnight). The next day, a band which appeared in 50 to 40% sucrose fraction was collected and diluted with PBS, and subjected to ultracentrifugation (35,000 rpm, 90 minutes) to obtain a pellet. This final pellet was suspended in a small volume of PBS to prepare purified Gag virus-like particles (VLP). The obtained VLPs were spherical particles with a diameter of 100 to 120 nm, and had an open circular or crescent-shaped electron-dense layer, which was characteristic to authentic immature HIV particles (Fig. ID).

### [Example 2] Confirmation of the presence of lipid bilayer membrane

It is known that the Gag protein in VLP which is completely enveloped by a lipid bilayer membrane is not digested by protease unless the lipid bilayer membrane is removed with a surfactant or organic solvent (Konvalinka, J. et al., 1995, Eur. J. Biochem. 228: 191-198; Spearman, P., and Ratner, L. 1996, J. Virol. 70: 8187-8194). In order to confirm that the VLP obtained by the present invention had the lipid bilayer membrane as an outer membrane, the VLP was subjected to 1% Triton X-100 treatment and trypsin digestion.

Triton X-100 was added to the purified VLP fraction to a final concentration of 1% in order to solubilize the lipid bilayer membrane. In the presence or absence of Triton X-100, trypsin was added to the VLP fraction at 1 mg/ml, and the Gag protein was digested at 30°C for 30 minutes.

The sample was analyzed by SDS-PAGE, followed by Western blotting. The anti-HIV-1 CA monoclonal antibody (Advanced Biotechnologies Inc.) was used for the detection of the Gag protein.

As a result, the Gag protein in VLP, which the lipid bilayer membrane was removed with Triton X-100, was completely degraded with trypsin, while the Gag protein in VLP which was not treated with Triton X-100 was not digested at all (Fig. 2). The results indicate that VLP which budded from the yeast spheroplast cell was completely enveloped by the lipid bilayer membrane.

### [Example 3] VLP production competence of truncation mutant of Gag

In the Gag protein expression in higher eukaryotic cells, it is known that, although the VLP production is not affected by the deletion of the p6-C terminus from the domain structure of the Gag protein (polyprotein of N terminus-MA-CA-p2-NC-p6-C terminus), VLP is not formed if the NC-p6-C terminus is deleted (Gheysen, D. et al., 1989, Cell 59: 103-112; Wang, C. T. et al., 1998, J. Virol. 72: 7950-7959). Whether these Gag mutants show similar assembly phenotypes when expressed in yeast was examined by expression of a wild type Gag protein (MA-CA-p2-NC-p6) and the truncation forms (MA-CA-p2-NC and MA-CA-p2) (Fig. 3A). The truncation forms were constructed by PCR. The VLP in the culture supernatant was subjected to equilibrium centrifugation in a 20-70% (w/v) sucrose gradient and detected by Western blotting using the relevant gradient fraction, which was fractioned from the bottom to the top.

The result showed that, although the expression levels of each Gag protein in the yeast cells were broadly equivalent, the VLPs were released into the culture supernatant in the case of the wild type and MA-CA-p2-NC, but, in contrast, in the case of MA-CA-p2, the VLP was not released to the culture supernatant (Figs. 3B, C).

This result indicates that the Gag region necessary for the VLP production of yeast is in agreement with that of the higher eukaryotic cell.

### [Example 4] The yield kinetics of VLP production with time

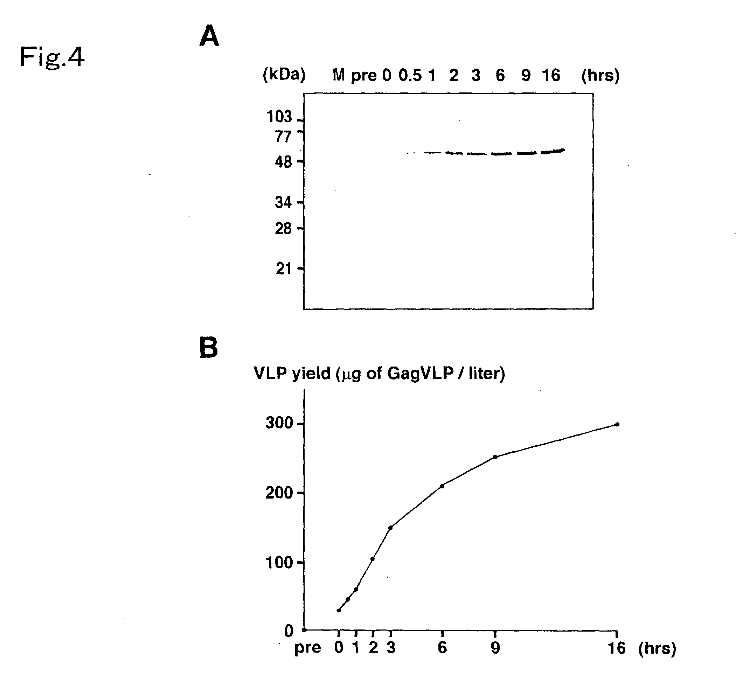

Changes in the level of VLP production with time were examined. The culture supernatant of the yeast spheroplasts expressing Gag protein was harvested at intervals, and the released VLP was purified to investigate the amount. As shown in Fig. 4A and B, the result showed that the VLP production started immediately after the initiation of spheroplast culture, and the level increased in a time-dependent manner. Based on simple quantitation by Western blotting using a series of 3-fold dilution and Coomassie brilliant blue staining, the level produced was estimated to be 300 µg of Gag VLP per liter of spheroplast culture by 16 hours (Fig. 4B).

### [Example 5] Production of VLP with addition of immunogenic epitope

It is known that the major immunogenic determinant of HIV is the V3 region of the spike protein gp120, in which a neutralizing epitope, Th epitope, and CTL epitope are present (Griffiths, J. C. et al., 1991, J. Virol. 65: 450-456). Thus, whether this V3 region could be incorporated into the VLP produced from yeast according to the present experiment or not was examined. A gene fragment encoding the V3 region was amplified by PCR and inserted in frame into downstream of cDNA encoding the wild type Gag protein. This expression plasmid was introduced into yeast, and VLP was purified from the culture supernatant of the spheroplast by equilibrium centrifugation in a 20-70% (w/v) sucrose gradient. The V3 antigen was detected when the purified VLP was analyzed by Western blotting using the anti-HIV-1 V3 monoclonal antibody (NEN-DuPont) having a neutralizing activity.

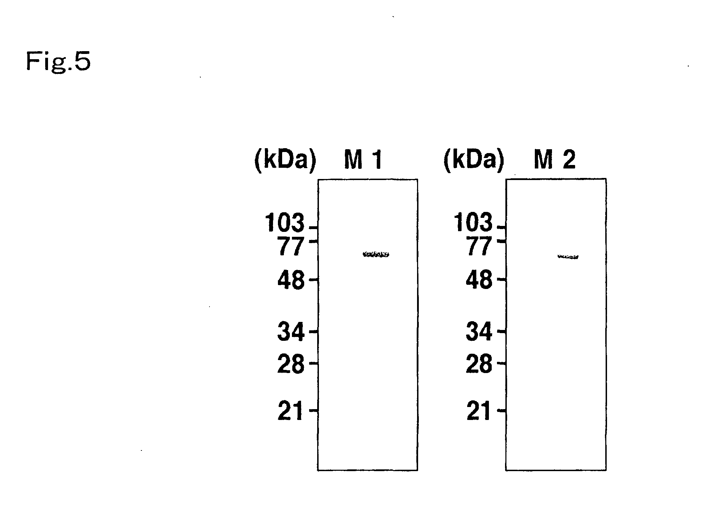

These results indicate that , the purified VLP were particles comprising the Gag-V3 fusion protein having the V3 region added to the carboxy terminus of the Gag protein, and this V3 region was found to react with the V3 monoclonal antibody having a neutralizing activity (Fig. 5).

### Industrial Applicability

As is apparent from the foregoing detailed description, the present invention provides virus-like particles enclosed with a lipid bilayer membrane derived from the eukaryotic microorganism as an outer membrane. These particles have various advantages: they can be produced in an easier manner and a significantly shorter period of time compared to the conventional process for producing virus-like particles; they are safe because they do not contain expression vector virus; and they are applicable to various applications.

All publications, patents and patent applications cited herein are incorporated herein by reference in their entirety.

## Claims

1. Virus-like particles having a lipid bilayer membrane derived from a eukaryotic microorganism as an outer membrane.

2. The virus-like particles according to claim 1, wherein the eukaryotic microorganism is yeast.

3. The virus-like particles according to claim 1 or 2, which have immunogenicity and do not produce infectious progeny viruses.

4. The virus-like particles according to any one of claims 1 to 3, wherein the immunogenicity derives from HIV.

5. A process for producing virus-like particles comprising:
(a) incorporating a gene encoding a protein essential for viral particle budding or a fragment thereof into a vector which can be expressed in a eukaryotic microorganism;
(b) transfecting the vector into the eukaryotic microorganism;
(c) culturing a transformed eukaryotic microorganism;
(d) removing a cell wall of the eukaryotic microorganism; and
(e) further culturing, followed by collection of a culture supernatant.

6. The process according to claim 5, wherein the gene or a fragment thereof comprises a gene encoding viral capsid protein or membrane/matrix protein or a fragment thereof.

7. The process according to claim 5, wherein the gene to be incorporated comprises a gene encoding a protein associated with immunogenicity or a fragment thereof.

8. The process according to any one of claims 5 to 7, wherein the eukaryotic microorganism is yeast.

9. The process according to any one of claims 5 to 8, wherein the virus is HIV.

10. A pharmaceutical composition, which comprises the virus-like particles according to any one of claims 1 to 4.

11. The pharmaceutical composition according to claim 10, which is a vaccine having immunogenicity derived from an incorporated virus gene.

12. The pharmaceutical composition according to claim 10, which comprises a virus-derived protein and at least one active ingredient.

13. The pharmaceutical composition according to claim 12, wherein the active ingredient is virus-derived nucleic acid, ribozyme, antisense nucleic acid, protein or a fragment thereof, or physiologically active protein or a fragment thereof.
